(19) Europäisches Patentamt
European Patent Office
Office européen des brevets

(11) Publication number: **0 409 559 B1**

(12) **EUROPEAN PATENT SPECIFICATION**

(45) Date of publication of patent specification: **11.01.95**

(51) Int. Cl.[6]: **A61K 31/20**

(21) Application number: **90307800.4**

(22) Date of filing: **17.07.90**

(54) **Pharmaceutical uses of fatty acids.**

(30) Priority: **21.07.89 GB 8916734**

(43) Date of publication of application:
**23.01.91 Bulletin 91/04**

(45) Publication of the grant of the patent:
**11.01.95 Bulletin 95/02**

(84) Designated Contracting States:
**AT BE CH DE DK ES FR GB GR IT LI LU NL SE**

(56) References cited:
**EP-A- 0 195 570**
**EP-A- 0 211 502**
**EP-A- 0 218 460**
**EP-A- 0 222 483**
**EP-A- 0 283 140**

(73) Proprietor: **SCOTIA HOLDINGS PLC**
**Efamol House**
**Woodbridge Meadows**
**Guildford**
**Surrey GU1 1BA (GB)**

(72) Inventor: **Horrobin, David Frederick**
**c/o Efamol House,**
**Woodbridge Meadows**
**Guildford,**
**Surrey GU1 1BA (GB)**

(74) Representative: **Sturt, Clifford Mark et al**
**J. MILLER & CO.**
**34 Bedford Row**
**Holborn**
**London WC1R 4JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to pharmaceutical and dietary uses of fatty acids.

GENERAL BACKGROUND - PGs

A great deal of attention has been paid in recent years to the formation and effects of prostaglandins (PGs). PGs of the 1-series, derived from DGLA, have desirable actions, PGs at the 2-series, formed from arachidonic acid, are of part desirable, part undesirable effect.

The outline of production of 1-series and 2-series PGs in the body is believed to be as shown in the following diagram:

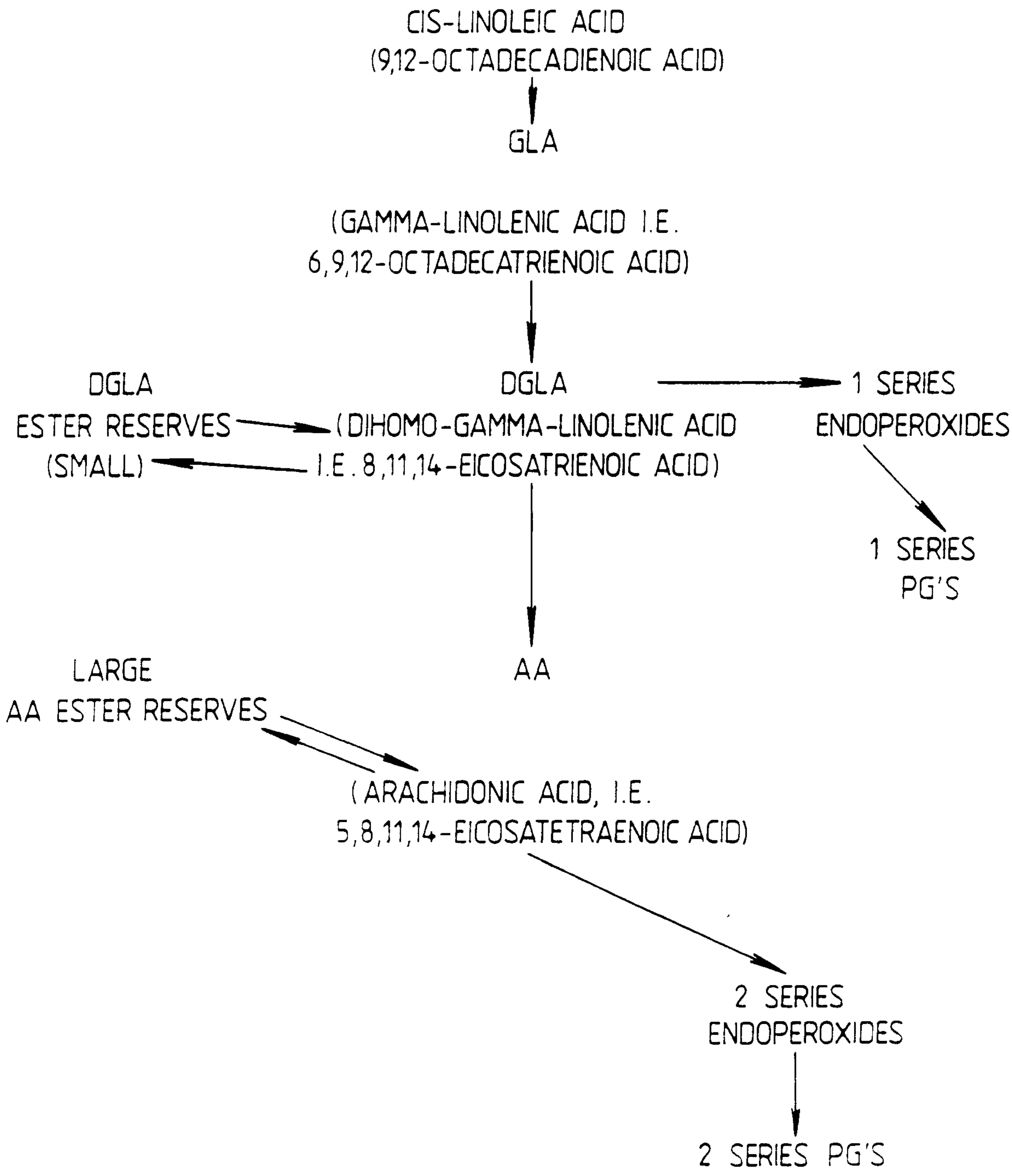

The broad outline of this pathway is well known, and it brings out clearly that a major function of essential fatty acids is to act as precursors for prostaglandins, 1-series PGs being formed from DGLA and 2-series PGs from arachidonic acid. Further, it has recently been found that the 22:4 n-6 acid produced from arachidonic acid gives rise to a series of homo-2-series PGs, though their importance is as yet unknown.

GENERAL BACKGROUND - FATTY ACIDS

A great deal of attention has also been paid to the essential fatty acids.

The pathways of metabolism of the n-6 essential fatty acids and the related n-3 acids sharing, it is believed, common enzymes in the two pathways, are:

| n-6 | | n-3 |
|---|---|---|
| 18:2 delta-9,12<br>(linoleic acid) | | 18:3 delta-9,12,15<br>(alpha-linolenic acid) |
| | ↓ delta-6 desaturase | |
| 18:3 delta-6,9,12<br>(gamma-linolenic acid) | | 18:4 delta-6,9,12,15 |
| | ↓ elongation | |
| 20:3 delta-8,11,14<br>(dihomo-gamma-linolenic acid) | | 20:4 delta-8,11,14,17 |
| | ↓ delta-5 desaturase | |
| 20:4 delta-5,8,11,14<br>(arachidonic acid) | | 20:5 delta-5,8,11,14,17 |
| | ↓ elongation | |
| 22:4 delta-7,10,13,16<br>(adrenic acid) | | 22:5 delta-7,10,13,16,19 |
| | ↓ delta-4 desaturase | |
| 22:5 delta-4,7,10,13,16 | | 22:6 delta-4,7,10,13,16,19 |

The pathways are not normally reversible nor, in man, are n-3 and n-6 series acids inter-convertible.

The acids, which naturally are of the all-cis configuration, are systematically named as derivatives of the corresponding octadecanoic, eicosanoic or docosanoic acids, e.g. delta-9,12-octadecadienoic acid or delta-4,7,10,13,16,19 docosahexaenoic acid, but numerical designation such as, correspondingly, 18:2 n-6 or 22:6 n-3 is convenient. Initials, for example, DHA for 22:6 n-3 (docosahexaenoic acid), are also used but do not serve when n-3 and n-6 acids of the same chain length and degree of unsaturation exist. Trivial names in more or less common use in the n-6 series are as shown. Of the n-3 series only 18:3 n-3 has a commonly used trivial name, alpha-linolenic acid. It was characterised earlier than gamma-linolenic acid and reference in the literature simply to linolenic acid, especially in the earlier literature, is to the alpha-acid.

DGLA is the key substance. GLA is almost completely and very rapidly converted in the body to DGLA and so for practical purposes the oral administration of DGLA and GLA amounts to the same thing. DGLA can be converted to a storage form or to PGs of the 1-series or, through arachidonic acid, to PGs of the 2-series.

## DEFICIENCIES AND BACKGROUND OF INVENTION

Considering dietary requirements, it is well known, for example, that linoleic acid cannot be made by the body and so must be taken in the diet. However, it has been generally thought that the body can metabolise linoleic acid to all the other n-6 acids and therefore that provided linoleic acid intake is adequate, no lack of the other n-6 acids will be found.

Occasionally, however, because of dietary deficiency, or failure of absorption due to loss of intestines or pancreatic or hepatic failure, or because of inadequate total parenteral nutrition, LA levels in blood are low and the levels of LA metabolites are also low. Much more common, however, is a normal or elevated level of linoleic acid, with a deficit of its metabolites, GLA, DGLA and AA. This is because the conversion of LA to GLA is normally rate-limiting and can be further reduced in a wide variety of situations leading to an inadequate flow of formation of GLA and the further metabolites. Situations in which AA is low include atopic disorders such as eczema, asthma and allergic rhinitis; elevation of cholesterol and increased risk of coronary heart disease; excess intake of alcohol and alcoholism; diabetes mellitus; inflammatory disorders such as rheumatoid arthritis and systemic sclerosis; vasospastic disorders such as Raynaud's syndrome; viral infections such as those with Epstein-Barr virus, respiratory tract viruses, AIDS, or the post-viral fatigue syndrome; warts; breast disorders and premenstrual syndrome; cancers of all types; prostate disorders and male and female infertility; stress associated disorders which produce increased levels of release of catecholamines and adrenal steroids.

Administration of either GLA or DGLA can readily correct the reduced levels of DGLA. However, because there is a second rate-limiting step between DGLA and AA, administration of GLA or DGLA often fails to bring AA levels back to normal. AA is available from the diet but normal dietary intakes often seem inadequate to restore plasma AA levels to normal. There is therefore a strong case for the direct administration of AA since AA is known to be a very important constituent of cell membranes.

Unfortunately, while AA is a vital component of membranes, it is also the precursor of a range of substances produced by cyclo-oxygenase and 5- and 12-lipoxygenase enzymes which are likely to have adverse effects including thrombosis, vascular spasm and inflammation. There is therefore considerable hesitation on the part of physicians about the value of administering AA because there is a fear of the occurrence of catastrophic side effects.

DGLA in contrast is converted to two metabolites which are highly desirable, PGE1 and 15-OH-DGLA. PGE1 is vasodilator, anti-thrombotic and anti-inflammatory and directly antagonizes many of the potential adverse effects of AA. Moreover, by stimulating the formation of cyclic AMP, PGE1 inhibits phospholipase A2 which releases free AA from membranes. PGE1 thus allows the incorporation of AA into membranes while inhibiting its release and is therefore likely to support the restoration of the normal structure of membranes which have been depleted of AA. Since only free AA can be converted to the harmful products, this retention of the AA in membranes will reduce the risk of adverse events. The other metabolite of DGLA, 15-OH-DGLA, is a potent inhibitor of the 5- and 12- lipoxygenase enzymes which convert AA to potentially harmful metabolites.

Free DGLA and DGLA salts dissociated at physiological pH values can be rapidly converted to PGE1 and 15- OH-DGLA. Esters, glycerides and other covalent derivatives of DGLA are less rapidly converted to the desirable metabolites, but might be expected to exert desirable effects under certain circumstances when co-administered with AA.

## THE INVENTION

The invention lies in the use of GLA and/or DGLA as such or in the form of a salt dissociated at physiological pH in the preparation of a medicament for use in reducing the risk of production of

undesirable metabolites from free bodily AA on administration of AA to a person suffering from a deficiency, thereof to correct such deficiency, wherein such deficiency does not arise as a side effect of diabetes mellitus or premenstrual syndrome.

Further, since n-3 EFAs also compete with AA for conversion to metabolites, and since the metabolites of the n-3 EFAs are consistently less harmful than the equivalent metabolites of AA, the GLA or DGLA can optionally be combined with an n-3 EFA, notably either eicosapentaenoic acid (EPA) or docosahexaenoic acid (DHA).

Thus the risk of administering AA in any form, whether as the free acid, mono- di- or triglyceride, ethyl, methyl or other ester, or any other physiologically equivalent derivative as discussed below, is reduced by supplying the AA together with free GLA/DGLA or an appropriate GLA/DGLA salt such as lithium, sodium or potassium DGLA. This combination can therefore be used to treat any condition in which blood or tissue AA levels are below normal except where the deficiency arises as a side effect of diabetes mellitus or premenstrual syndrome. In particular, the conditions mentioned above may be treated.

DGLA given as such or produced from GLA produces the desired effect in at least three ways:

1. DGLA is converted to PGE1 which directly antagonizes the two most dangerous effects of AA, excess platelet aggregation and vasoconstriction, produced by a number of AA metabolites but notably and most potently by thromboxane A2.
2. Much of the AA administered is rapidly incorporated into phospholipids in cell membranes where it exerts a desirable effect by improving membrane fluidity and flexibility. While the increased level of AA in cell membranes would mean that more AA was available to be released from those membranes by phospholipase A2 (which makes AA available for conversion to its metabolites), PGE1 raises levels of cyclic AMP which in turn inhibits phospholipase A2. The DGLA administered therefore helps to ensure that the administered AA remains in the membranes where it is useful and is not released by phospholipase A2 to the free acid form, readily converted to potentially harmful metabolites such as thromboxane A2 and the various leukotrienes.
3. Free AA is converted by cyclo-oxygenase to a range of prostaglandins and related substances, including thromboxane A2. It is also converted by 5-lipoxygenase, which forms a variety of pro-inflammatory agents including the leukotrienes, and to 12- lipoxygenase which leads to the formation of other pro- inflammatory substances, notably 12-hydroxyeicosatetraenoic acid (12-HETE). However DGLA leads to the formation of 15-OH-DGLA which inhibits both the 5- and 12- lipoxygenases.

## FORMS OF AA AND OTHER ACIDS

The AA and n-3 acids if present may be used as such or as pharmaceutically acceptable and physiologically equivalent derivatives as, for example, salts, amides, esters including glyceride esters and alkyl (e.g. C1 to C4) esters, and phospholipids, and references to the acids herein whether in the claims or elsewhere are to be taken as including reference to them when in the form of such derivatives. Equivalence is demonstrated by entry into the pathway quoted herein, as evidenced by effects corresponding to those of the acid itself or its natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al p 23, 'Analysis of Lipids and Lipoproteins' Ed Perkins, American Oil Chemists Society, Champaign, Illinois, U.S.A.

In outline the method is suitably that plasma samples (1ml) are extracted with chloroform:methanol (2:1). The extract is filtered through sodium sulphate, evaporated to dryness, and taken up in 0.5ml chloroform: methanol. The lipid fractions are separated by thin layer chromatography on silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot column packed with 10% silar on chromosorb WAW 106/230. The carrier gas is helium (30ml/min). Oven temperature is programmed to rise from 165° to 190° at 2°C/min. Detector temperature is 220° and injector temperature 200oC. Retention times and peak areas are automatically computed by Hewlett-Packard Level 4 integrator. Peaks are identified by comparison with standard fatty acid methyl esters.

## DIETARY COMPOSITIONS

The invention is chiefly described in terms of methods of treatment and pharmaceutical compositions, but it will be understood that the gamma-linolenic and other acids, being in the nature of dietary

supplements, could be incorporated in a dietary margarine or other foodstuff which would then constitute a medicament as referred to herein.

## RANGES AND DOSES

Effective ranges of AA to GLA/DGLA ratio are wide, but desirably the range is from 10:1 to 1:10 and preferably from 4:1 to 1:4, by weight.

The dose of AA in the above mixture is desirably 10 mg to 10 g and preferably 100 mg to 3 g per day. Further, where salts are present, the salt-forming moieties should be in amounts themselves physiologically acceptable, for example sodium or potassium up to 4 g/day and lithium up to 400 mg/day.

## SOURCES OF GAMMA-LINOLENIC AND OTHER ACIDS

Known natural sources of oils having a high gamma-linolenic acid content include the seed of Evening Primrose species such as Oenothera biennis L and Oenothera lamarckiana, the oil extract therefrom containing gamma-linolenic acid (about 8%) and linoleic acid (about 72%) in the form of their glycerides together with other glycerides (percentages based on total fatty acids). Other sources of gamma-linolenic acids are Borage species such as Borage officinalis which, though current yield per acre is low, provide a richer source of gamma-linolenic acid than Oenothera oil. Recent studies on fungi which can be cultivated by fermentation promise a fungal oil source.

The oil is extracted from seed by one of the conventional methods of extraction such as cold pressure, screw pressure after partially cooking the seed, or solvent extraction.

Fractionation of a typical sample of this oil in the form of methyl esters shows the relative proportions:

| | |
|---|---|
| Palmitate | 6.15 |
| Stearate | 1.6 |
| Oleate | 10.15 |
| Linoleate | 72.6 |
| Gamma-linolenate | 8.9 |

As preservative, alpha-tocopherol is added to the oil in a concentration 0.1%.

The seed oil extracts referred to above can be used as such or can, for example, if desired, be fractionated to yield an oily composition containing the triglycerides of gamma-linolenic and linoleic as the main fatty acid components, the gamma-linolenic acid content being if desired a major proportion. Seed oil extracts appear to have a stabilising effect upon dihomo-gamma-linolenic acid if present.

## SOURCES OF OTHER ACIDS

Of the acids other than GLA, AA is readily available from animal sources, or from fermentation of certain fungi such as Mortierella spp or by chemical synthesis. Natural sources of DGLA are limited but there are fungal sources for example Mortierella alpina (Shimizu et al, JAOCS 66 No 2 pp 237-241 February 1989). The acid can be isolated from these sources by, for example, saponification under mild non-oxidising conditions followed by preparative gas liquid chromatography. Synthesis of the DGLA is difficult but not impossible and provides another source.

## PHARMACEUTICAL PRESENTATION

The compositions according to the invention are conveniently in a form suitable for oral, rectal or parenteral administration in a suitable pharmaceutical vehicle, as discussed in detail for example in Williams British Patent Specification No. 1,082,624, to which reference may be made, and in any case very well known generally for any particular kind of preparation. Thus, for example, tablets, capsules, ingestible liquid or powder preparations can be prepared as required, and topical preparations also when the gamma-linolenic acid or other acids are absorbed through the skin. Injectable solutions of hydrolysed Oenothera oil may be prepared using albumin to solubilise the free acid.

Advantageously, a preservative is incorporated into the preparations. Alpha-tocopherol in concentration of about 0.1% by weight has been found suitable for the purpose.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

EXAMPLES

Soft or hard gelatine capsules with or without enteric coating, made by conventional methods, and other preparations, are administered to counter low AA levels in the conditions referred to herein, as follows:

1. Soft gelatine capsules containing 200mg ethyl-AA and 200mg free DGLA, six daily.
2. Hard gelatine capsules containing 300mg AA in triglyceride form and 100mg of a lithium, sodium or potassium salt of DGLA, three daily.
3. Hard gelatine, enteric coated capsules containing 100mg of AA free acid and 200mg of DGLA free acid, twelve daily.
4. A solution for enteral nutrition containing 100mg/100ml of AA as ethyl ester, triglyceride or sodium salt, + 25mg of DGLA sodium salt and 25mg of DGLA lithium salt, 100 ml daily.
5. An emulsion for parenteral administration containing 100mg/100ml ethyl-AA, plus 50mg of lithium, sodium or potassium-DGLA, 100 ml daily.
6. A solution for topical administration containing 30mg/ml of the lithium, sodium or potassium salt of DGLA and 20mg/ml of the lithium, sodium or potassium salt of AA, 20 ml applied three times daily.
7. A cream for topical administration containing 50mg/ml ethyl AA and 50mg/ml sodium-DGLA, 10 ml applied three times daily.

**Claims**

**1.** Use of GLA and/or DGLA as such or in the form of a salt dissociated at physiological pH in the preparation of a medicament for use in reducing the risk of production of undesirable metabolites from free bodily AA on administration of AA to a person suffering from a deficiency thereof to correct such deficiency, wherein such deficiency does not arise as a side effect of diabetes mellitus or premenstrual syndrome.

**2.** A use according to claim 1 wherein said medicament further contains the AA to be administered.

**3.** A use according to claim 1 or 2, wherein said medicament further contains EPA, DHA or other n-3 series essential fatty acid.

**4.** Use of GLA and/or DGLA in the form of esters, glycerides and other covalent derivatives together with AA in the preparation of a medicament for use in reducing the risk of production of undesirable metabolites from free bodily AA on administration of AA to a person suffering from a deficiency thereof to correct such deficiency, wherein such deficiency does not arise as a side effect of diabetes mellitus or premenstrual syndrome.

**Patentansprüche**

**1.** Verwendung von GLA und/oder DGLA als solche oder in Form eines Salzes, dissoziiert bei einem physiologischen pH, bei der Zubereitung eines Medikamentes zur Verwendung für die Reduzierung des Risikos der Bildung von unerwünschten Metaboliten von Freikörper AA bei der Eingabe von AA zu einer Person, die an einem Mangel daran leidet, um derart diesen Mangel zu korrigieren, wobei ein derartiger Mangel nicht aufgrund eines Nebeneffektes von Diabetes Mellitus oder des prämenstruellen Syndromes entsteht.

**2.** Verwendung gemäß Anspruch 1, wobei besagtes Medikament weiterhin die einzunehmende AA enthält.

**3.** Verwendung gemäß Anspruch 1 oder 2, worin besagtes Medikament weiterhin EPA, DHA oder andere n-3 Serien essentielle Fettsäure enthält.

4. Verwendung von GLA und/oder DGLA in der Form von Estern, Glyceriden und anderen kovalenten Derivaten zusammen mit AA bei der Zubereitung eines Medikamentes zur Verwendung für die Reduzierung des Risikos der Bildung von unverwünschten Metaboliten von Freikörper AA bei der Eingabe von AA zu einer Person, die an einem Mangel davon leidet, um einen derartigen Mangel zu korrigieren, wobei ein derartiger Mangel nicht aufgrund eines Nebeneffektes von Diabetes Mellitus oder des prämenstruellen Syndroms entsteht.

**Revendications**

1. Utilisation d'acide gamma-linolénique (GLA) et/ou d'acide dihomo-gamma-linolénique (DGLA), en l'état ou sous forme d'un sel, dissocié à un pH physiologique, pour préparer un médicament destiné à être utilisé dans la réduction du risque de production de métabolites indésirables à partir d'acide arachidonique libre (AA), par administration d'AA à une personne souffrant d'une déficience en AA, pour corriger cette déficience, cette déficience n'apparaissant pas sous forme d'un effet secondaire du diabète sucré ou du syndrome prémenstruel.

2. Utilisation selon la revendication 1, dans laquelle le médicament contient en outre l'AA à administrer.

3. Utilisation selon la revendication 1 ou 2, dans laquelle le médicament contient en outre de l'acide éicosapentaénoïque (EPA), de l'acide docosahexaénoïque (DHA), ou un autre acide gras essentiel de la série n-3.

4. Utilisation du GLA et/ou du DGLA sous forme d'esters, de glycérides ou d'autres dérivés covalents, en même temps que de l'AA, pour préparer un médicament destiné à être utilisé dans la réduction du risque de réduction de métabolites indésirables à partir d'AA libre, lors de l'administration d'AA à une personne souffrant d'une déficience en AA, pour corriger cette déficience, cette déficience n'apparaissant pas sous forme d'un effet secondaire du diabète sucré ou du syndrome prémenstruel.